Europäisches Patentamt

European Patent Office

Office européen des brevets

(11) Publication number: **0 169 704**
**B1**

# EUROPEAN PATENT SPECIFICATION

(45) Date of publication of patent specification: **15.11.89**

(51) Int. Cl.⁴: **A 61 M 5/14, A 61 M 25/00**

(21) Application number: **85305093.8**

(22) Date of filing: **17.07.85**

(54) **Intravascular device including a clip for use in preventing needle retraction.**

(30) Priority: **21.07.84 GB 8418655**

(43) Date of publication of application:
**29.01.86 Bulletin 86/05**

(45) Publication of the grant of the patent:
**15.11.89 Bulletin 89/46**

(84) Designated Contracting States:
**AT BE CH DE FR IT LI LU NL SE**

(56) References cited:
**GB-A-2 063 679**
**US-A-4 311 137**
**US-A-4 362 156**

(73) Proprietor: **H.G. Wallace Limited**
**Whitehall Road**
**Colchester Essex CO2 8JH (GB)**

(72) Inventor: **Wallace, Henry George**
**Treetops First Avenue**
**Frinton-on-Sea, Essex (GB)**

(74) Representative: **Green, Alan James et al**
**SANDERSON & CO. European Patent Attorneys**
**34, East Stockwell Street**
**Colchester Essex CO1 1ST (GB)**

Courier Press, Leamington Spa, England.

## Description

The present invention relates to the prevention of needle retraction in intravascular devices and, in particular, to devices including a clip for use in preventing needle retraction.

Intravascular devices are commonly used for supplying fluid to a patient or for the withdrawal or monitoring of blood. Thus, for example, they may be used to supply a drug on a continuous basis, for blood pressure measurement, or for extraction and return of blood in haemodialysis.

In use of such devices it is customary to introduce a plastics cannula into a vein or artery by means of a metal introducing needle. The introducing needle is incorporated within the lumen of the cannula·so as to act as means to effect tissue puncture and to introduce the cannula. After such puncture the needle is withdrawn leaving the cannula in situ to be coupled to suitable equipment for the required treatment or procedure.

In such intravascular devices the hollow introducing needle may be bonded or fixed by some other method to a hub or coupling member serving as a finger grip, and at its sharpened distal end the needle protrudes beyond the cannula lumen. Tissue puncture is thus effected by that sharpened distal end, and permits the cannula to follow the needle until it too progresses to a second puncture of the blood vessel. When entry to the blood vessel is verified by a "flashback" of blood through the hollow needle to the transparent needle hub, the needle is withdrawn leaving the cannula disposed within the blood vessel to serve as the desired conduit into the patient's bloodstream.

With such devices, a problem arises in use when the needle hub cannot be used as a finger grip. This may be the case, for example, when the needle and cannula are too long or the shape or constitution of the device makes gripping the needle via the needle hub cumbersome and/or unstable. In such cases the operative may have to transfer his or her grip to a more suitable part of the device, for example, nearer to the needle tip to reduce the effective needle length. However, when the grip is transferred from the needle hub, for example, to the hub portion attached to the cannula, typically where that hub portion is of elastomeric material e.g. polyurethane, pressure on the needle tip caused by tissue resistance can overcome the thus reduced gripping force and, in turn, can cause the needle to retract within the lumen of the cannula. Thus, the sharp needle tip can be prevented from functioning in its necessary role of effecting initial tissue puncture.

In U.S. Specification No. 4,314,555 there is described an intravascular catheter assembly including a location bar to restrain forward movement of the needle relative to the cannula, and to define the extent to which the needle is inserted into the cannula, so as to cause the distal end of the needle slightly to protrude from the distal end of the cannula. However, the disclosed assembly includes no means to prevent any rearward movement of the needle leading to needle retraction within the cannula.

In British Specification No. 1,590,796 there is described needle restraining means to restrain relative axial movement in either direction between the cannula and the introducing needle. The disclosed restraining means fits over substantially the entire length of the flexible sleeve portion of the disclosed catheter assembly and connects said portion to the needle hub portion to restrain both forward and rearward movement of the needle (and thus to prevent needle retraction within the cannula). The restraining means also includes means enabling the selective release of the needle assembly to permit withdrawal of the needle assembly after venipuncture.

However, in the specific embodiments disclosed in that British specification, the needle restraining means comprises various collar or like arrangements wholly surrounding the intravascular device. For the majority of such specific embodiments the restraining means must be released along an upper portion e.g. released by a tear strip portion running along the length of the restraining means, and then removed from beneath the intravascular device, i.e. from between the device and the adjacent portion of the patient's body. However, to effect such removal without disturbing the device, is in most circumstances extremely difficult, if not impossible. While it is perhaps possible with care to release the restraining means without disturbing the device, also to remove the restraining means without disturbance would appear to be impossible in most circumstances. Furthermore, while there is also disclosed an additional embodiment wherein a needle restraining collar is rotated to effect release, in that case the operation of releasing the restraining means would appear to cause undesirable disturbance of the in situ device.

Other prior art devices are shown in U.S. Specifications Nos. 4,311,137 and 4,362,156 as well as in British Specification No. 2,063,679. In the latter, in particular in Figures 13 and 14, there is shown a latching mechanism which includes a pair of arms which engage within a circular groove 88 in the catheter hub portion 14.

In the present invention we seek to provide for an intravascular device which comprises a catheter suitable for haemodialysis, a clip to effect a relatively simple and temporary locking of the needle relative to the cannula in order to avoid the serious inconvenience of needle retraction, which clip can easily be released without serious disturbance of the in situ device carrying the needle.

Accordingly, the present invention provides an intravascular device, which device comprises a catheter suitable for haemodialysis, the catheter having a cannula and a hub portion including a first section of larger diameter than an adjacent second section to provide a shoulder in the hub portion facing the cannula, the second section being elongate and of substantially constant outside diameter, and the device housing a tissue puncture needle within the cannula lumen and the hub portion, the device including a clip for use in preventing retraction of the tip of the tissue puncture needle within the cannula lumen of the device, which clip comprises a body member having at or adjacent one end a first portion comprising means such as a pair of opposed depending arms associated with an assembly comprising said tissue puncture needle at a portion of the assembly proximal to the hub and remote from the needle tip and, spaced from the first clip portion, a second clip portion engageable with the second section of the hub portion to lock the needle relative to the cannula against movement permitting said retraction, the second clip portion being releasable by movement away from the device which does not necessitate movement of the device away from the patient's body once puncture has been effected, and the second clip portion comprising a pair of opposed depending arms formed so as releasably to grip the second section of the hub portion of the catheter immediately in front of the shoulder portion and to abut the shoulder portion to prevent needle retraction.

The device of the present invention may be any intravascular device which comprises a catheter suitable for haemodialysis in which the hub portion includes a first section or larger diameter than an adjacent second section to provide a shoulder in the hub portion facing the cannula with which the second portion of the clip can engage. For example, the clip is particularly useful in conjunction with haemodialysis catheters such as those described in British Patent Specification No. 2,075,347B.

Thus, in one particular aspect the invention provides a device suitable for haemodialysis as defined above and comprising a cannula portion and a hub portion integral with connecting means for providing a secure, lockable connection to a blood line, said hub portion being of resilient material whereby its lumen may be closed by externally applied pressure both before and after connection of the hub portion to a blood line.

Preferably, such a device of that one aspect is a device having a Y-shaped hub portion, each branch of the hub portion being integral with means for providing a secure, lockable connection to a blood line and the lumen of each branch being closable by externally applied pressure before or after connection of said branch to a blood line.

In the device of the invention, the first clip portion may be formed separately of or integrally with the needle assembly. Thus, for example, the first clip portion may be integral with or merely engageable with the needle assembly.

Furthermore, the second clip portion may be engageable with the device in any manner which permits releasable engagement, and release of the clip by movement thereof away from the device in a manner such that there is no necessity to move the device away from the patient's body. Preferably, however, the first and second portions of the clip are arranged so that any second portion or portions can be released without detachment of the first portion from the needle assembly.

In one preferred embodiment of the invention the device comprises a cannula and a hub portion having at the end of the hub portion remote from the cannula means for connection to a lock connector, typically a luer lock or Record lock connector. Typically, the luer lock or like lock connection means may comprise a pair of lugs preferably of wedge-shaped form, upstanding from the outer surface of the proximal end of the hub portion. Such lugs are formed so that one surface faces towards the needle tip, and that surface is preferably at a helical angle corresponding to the helical angle of the luer lock or like lock connector.

Preferably, in the device of the invention the needle assembly is formed to include a pair of trunnion pins upstanding on either side of a portion of the assembly serving as a finger grip. More preferably, the clip is formed with a first pair of opposed arms each including an aperture so that they can be sprung over the trunnion pins and the clip thereby pivotably mounted on the needle assembly finger grip portion. By such means, the clip can be mounted so that it may be pivoted into and out of a locking position, and the remaining pair of arms may be formed so as releasably to grip the hub portion attached to the cannula immediately in front of the shoulder portion formed in the hub portion and facing the cannula, and to abut the shoulder portion to prevent needle retraction. Typically, such a shoulder portion will be formed between the cannula and any connection means, e.g. luer, luer lock or like lock connection means, at the proximal end of the hub portion.

Preferably, the trunnion pins are formed at or adjacent the end of the said finger grip portion of the needle assembly nearer the needle tip. Preferably also, at least the finger grip portion of the needle assembly containing the catheter lumen adjacent the non-distal end is transparent so as readily to show "flashback". In addition, it is preferred that the device is one having at or adjacent the proximal end of the needle assembly means for connection to a lock connector which comprise a pair of lugs upstanding from the outer surface of the proximal end of the needle assembly with one surface thereof facing towards the needle tip, preferably to provide a luer lock connection.

The clip used in the device of the invention may be formed of any suitable resilient material. Preferably, however, the clip is formed of a synthetic plastics material, for example, nylon or polypropylene. Furthermore, the clip used in the device preferably may be one which includes a finger grippable nose portion extending towards the needle tip away from the second clip portion, the nose portion having ridges to prevent slippage when gripped by the tip of a finger.

The device of the invention will now be described by way of example with reference to the accompanying drawings in which:

Figure 1 is a side view of one form of clip used in a device in accordance with the invention;

Figure 2 is a front view of the clip of Figure 1; and

Figure 3 shows the clip of Figure 1 as part of an intravascular device assembly.

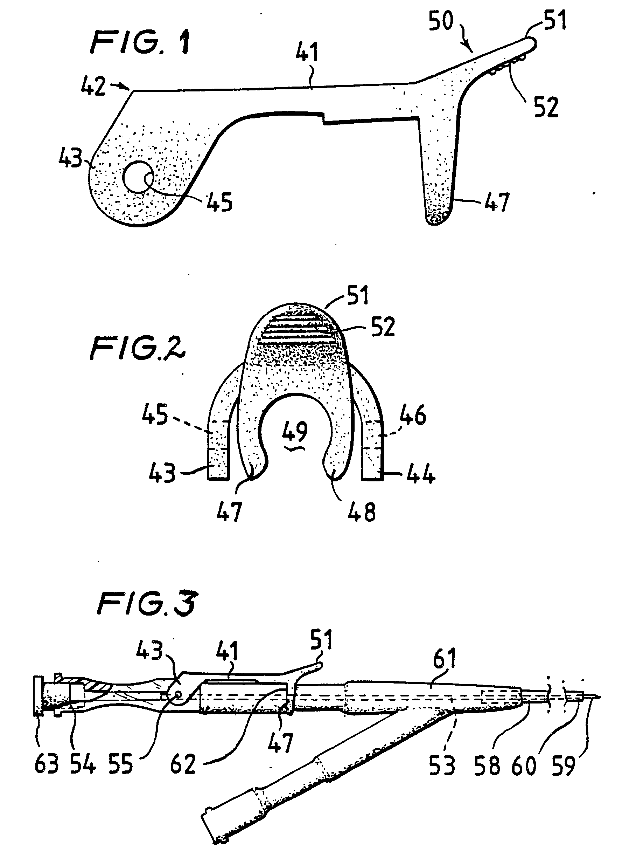

Referring to Figures 1 and 2, the clip there shown comprises a body member 41 having at one end 42 a first pair of opposed arms 43 and 44. The arms 43 and 44 depend downwardly from the body member 41 and include through apertures 45 and 46 respectively.

Remote from arms 43 and 44 the body member 41 includes a second pair of opposed arms 47 and 48 between them defining a recess 49 within which a hub portion of an intravascular device can be accommodated. In addition, at its end 50 beyond arms 47 and 48 the body member 41 includes a finger grippable nose portion 51 having ridges 52 to prevent slippage when gripped by a finger tip. The clip may be formed of a resilient plastics material such as nylon or polypropylene.

The clip of Figures 1 and 2 is added for use with a needle assembly as shown in Figure 3. As there shown, the needle assembly comprises a needle 53 and a transparent needle hub 54 including on each side trunnion pins 55 (only one shown). In use the first pair of opposed arms 43 and 44 are sprung over the trunnion pins 55 so that the pins are accommodated in apertures 45 and 46 respectively, whereby the clip is pivotably mounted on the needle hub 54. When thus mounted on the needle hub, and with the needle 53 disposed within the lumen of cannula 58, whereby sharpened needle tip 59 protrudes beyond end 60 of the cannula 58, the clip can be depressed onto hub portion 61 attached to the cannula 58, so that arms 47 and 48 move apart and the hub portion 61 is sprung into the recess 49. Then, by virtue of the presence of shoulder 62 on hub portion 61, abutting surfaces of the shoulder 62 and the arms 47 and 48 lock the needle 53 relative to the cannula 58 to prevent rearward movement which might permit tip 59 to retract within the lumen.

Once "flashback" within needle hub 54 is seen, and tissue puncture is thereby confirmed, the clip may be released by applying finger pressure to nose portion 51 at ridges 52 to release hub portion 61 from recess 49. Once the clip is thus released the needle may be withdrawn to permit the desired connection to hub portion 61 to be effected by removal of plug 63.

The hub portion 61 may be adapted to accommodate a luer connector or may include luer lock or like lock lugs (not shown) at its proximal end beyond the shoulder 62. Also, the hub portion may include a self-sealing rubber or like seal (not shown) either within the hub portion or adjacent its proximal end to permit needle withdrawal without blood spillage.

As will be appreciated from the above specific description, the present invention provides a device including a clip which can prevent needle retraction while at the same time being releasable with minimum disturbance to the in situ device. Also, because the clip as described is both releasable from and returnable to the locking configuration even while the device is in use, any initial error in effecting puncture can be adjusted by releasing the clip, partially withdrawing the needle, returning the needle, and relocking the clip.

## Claims

1. An intravascular device, which device comprises a catheter suitable for haemodialysis, the catheter having a cannula (58) and a hub portion (61) including a first section of larger diameter than an adjacent second section to provide a shoulder (62) in the hub portion facing the cannula, the second section being elongate and of substantially constant outside diameter, and the device housing a tissue puncture needle (53) within the cannula lumen and the hub portion, the device including a clip for use in preventing retraction of the tip of the tissue puncture needle within the cannula lumen of the device, which clip comprises a body member (41) having at or adjacent one end (42) a first portion comprising means such as a pair of opposed depending arms (43, 44) associated with an assembly comprising said tissue puncture needle at a portion of the assembly proximal to the hub and remote from the needle tip and spaced from the first clip portion, a second clip portion engageable with the second section of the hub portion to lock the needle relative to the cannula against movement permitting said retraction, the second clip portion being releasable by movement away from the device which does not necessitate movement of the device away from the patient's body once puncture has been effected, and the second portion comprising a pair of opposed depending arms (47, 48) formed so as releasably to grip the second section of the hub portion of the catheter immediately in front of the shoulder portion and to abut the shoulder portion to prevent needle retraction.

2. A device according to claim 1, wherein the first and second portions of the clip are arranged so that the clip can be released without detachment of the first portion from the needle assembly.

3. A device according to claim 1 or claim 2, wherein the clip is formed separately of the needle assembly.

4. A device according to any one of the preceding claims having at the end of the hub portion remote from the cannula means for connection to a lock connector.

5. A device according to claim 4, wherein the lock connection means comprise a pair of lugs upstanding from the outer surface of the proximal end of the hub portion with one surface thereof facing towards the needle tip, preferably to provide a luer lock connection.

6. A device according to any one of the preceding claims, wherein the needle assembly is formed to include a pair of trunnion pins (55) upstanding on either side of a portion of the assembly serving as a finger grip, and preferably wherein the clip is formed with a first pair of opposed arms each including an aperture so that they can be sprung over the trunnion pins and the clip thereby pivotably mounted on the needle assembly hub portion.

7. A device according to claim 6, wherein the pins are formed at or adjacent the end of the said portion of the needle assembly nearer the needle tip.

8. A device according to claim 7, wherein at least the portion of the needle assembly containing the catheter lumen adjacent the non-distal end is transparent so as readily to show flashback.

9. A device according to any one of the preceding claims having at or adjacent the proximal end of the needle assembly means for connection to a lock connector which comprise a pair of lugs upstanding from the outer surface of the proximal end of the needle assembly with one surface thereof facing towards the needle tip, preferably to provide a luer lock connection.

10. A device according to any one of the preceding claims, wherein the clip includes a finger grippable nose portion (51) extending towards the needle tip away from the second clip portion, the nose portion having ridges (52) to prevent slippage when gripped by the tip of a finger.

11. A device according to any one of the preceding claims wherein the hub portion is integral with connecting means for providing a secure, lockable connection to a blood line, said hub portion being of resilient material whereby its lumen may be closed by externally applied pressure both before and after connection of the hub portion to a blood line.

12. A device according to claim 11 having a Y-shaped hub portion, each branch of the hub portion being integral with means for providing a secure, lockable connection to a blood line and the lumen of each branch being closable by externally applied pressure before or after connection of said branch to a blood line.

**Patentansprüche**

1. Eine intravaskuläre Vorrichtung, dadurch gekennzeichnet, dass diese Vorrichtung ein für die Hämodialyse geeigneter Katheter ist, der eine Kanüle (58) und ein Verbindungsstück (61) aufweist, das einen ersten Teil mit einem grösseren Durchmesser als der angrenzende Teil umfasst, sodass im Verbindungsstück gegen die Kanüle hin eine Schulter (62) entsteht, wobei der zweite Teil verlängert ist und einen weitgehend gleichbleibenden Durchmesser aufweist, und dass in der Vorrichtung im Hohlraum der Kanüle und des Verbindungsstückes eine Gewebe-Punktionsnadel eingebaut ist, dass die Vorrichtung mit einem Halter versehen ist, der dazu gebraucht wird, den Rückzug der Spitze der Gewebe-Punktionsnadel in den Hohlraum der Kanüle der Vorrichtung zu verhindern, dass dieser Halter einen beweglichen Bestandteil (41) umfasst, der am oder anschliessend an ein Ende (42) des ersten Teiles ein Element von der Art eines Paares gegenüberliegender, herabgebogener Arme (43, 44) umfasst, die mit einem zusammengebauten Teil verbunden sind, bestehend aus der besagten Gewebepunktionsnadel beim zusammengebauten Teil proximal der Buchse und entfernt von der Nadelspitze, und aus einem zweiten Halterteil mit einem Zwischenraum vom ersten Halterteil, der beim zweiten Abschnitt des Verbindungsstückes eingehakt werden kann, um die Nadel gegenüber der Kanüle gegen Bewegung, die den besagten Rückzug erlaubt, zu sichern, wobei der zweite Teil des Halters durch Wegklappen von der Vorrichtung lösbar bleibt, sodass nach erfolgter Punktion keine Wegnahme der Vorrichtung vom Körper des Patienten nötig ist, und einem zweiten Teil des Halters, bestehend aus einem Paar gegenüberliegender, herabgebogener Arme (47, 48), die so ausgebildet sind, dass sie lösbar in den zweiten Teil des Verbindungsstückes des Katheters unmittelbar vor dem Schulter-Abschnitt und anstossend an den Schulterabschnitt eingreifen, um den Rückzug der Nadel zu verhindern.

2. Eine Vorrichtung nach Anspruch 1, dadurch gekennzeichnet, dass der erste und zweite Teil des Halters so angeordnet sind, dass der Halter ohne Abtrennung des ersten Abschnittes vom zusammengebauten Teil mit der Nadel gelöst werden kann.

3. Eine Vorrichtung nach Anspruch 1 oder 2, dadurch gekennzeichnet, dass der Halter vom zusammengebauten Teil mit der Nadel getrennt ausgebildet ist.

4. Eine Vorrichtung nach einem der vorhergehenden Ansprüche, dadurch gekennzeichnet, dass sie am Ende des Verbindungsstückes von der Kanüle entfernt Elemente für den Anschluss an einen verriegelbaren Verbinder aufweist.

5. Eine Vorrichtung nach Anspruch 4, dadurch gekennzeichnet, dass die Elemente des verriegelbaren Verbinders ein Paar von der äusseren Fläche des proximalen Endes des Verbindungsstückes aufstehende Nasen aufweist, deren eine Fläche gegen die Nadelspitze schaut, vorzugsweise um eine VerschlussVerbindung nach Luer zu liefern.

6. Eine Vorrichtung gemäss einem der vorhergehenden Ansprüche, dadurch gekennzeichnet, dass der Nadelzusammenbau so ausgebildet ist, dass er ein Paar als Drehzapfen ausgebildete Nadeln (55) aufweist, die auf jeder Seite des zusammengebauten Teiles, der als Fingergriff dient, aufstehen, und der Halter vorzugsweise aus einem ersten Paar von entgegengesetzten Armen ausgebildet ist, von denen jeder eine Öffnung enthält, sodass sie über die als Drehzapfen ausgebildeten Nadeln eingehängt werden können, und dabei der Halter schwenkbar auf dem Verbindungsstück des zusammengebauten Teiles mit der Nadel angebracht wird.

7. Eine Vorrichtung nach Anspruch 6, dadurch gekennzeichnet, dass die Nadeln am oder anschliessend an das Ende des besagten Teiles des zusammengebauten Teiles mit der Nadel näher gegen die Nadelspitze hin ausgebildet sind.

8. Eine Vorrichtung nach Anspruch 7, dadurch gekennzeichnet, dass wenigstens der Teil des Nadel-Zusammenbaus, welcher das Katheter-Lumen anschliessend an das nicht distale Ende enthält durchsichtig ist, sodass ein Rückfluss rasch angezeigt wird.

9. Eine Vorrichtung nach einem der vorhergehenden Ansprüche, dadurch gekennzeichnet, dass sie am oder anschliessend an das proximale Ende des zusammengebauten Teiles mit der Nadel Elemente für den Anschluss an einen verriegelbaren Verbinder aufweist, welche ein Paar Nasen enthalten, welche von der äusseren Fläche des proximalen Endes des zusammengebauten Teils mit der Nadel aufstehen, und mit einer seiner Flächen gegen die Nadelspitze schauend, vorzugsweise um eine Verschluss-Verbindung nach Luer zu liefern.

10. Eine Vorrichtung nach einem der vorhergehenden Ansprüche, dadurch gekennzeichnet, dass der Halter einen mit den Fingern ergreifbaren Nasenteil (51) aufweist, der gegen die Nadelspitze hin vom zweiten Halterteil absteht, wobei der Nasenteil Rippen (52) aufweist, um ein Gleiten zu verhindern, wenn er mit den Fingern ergriffen wird.

11. Eine Vorrichtung nach einem der vorstehenden Ansprüche, dadurch gekennzeichnet, dass darin das Verbindungsstück an die Verbindungs-Elemente angebaut ist, um eine sichere, verriegelbare Verbindung zu einer Blutleitung zu liefern, wobei besagtes Verbindungsstück aus elastischem Material besteht, sodass sein Lumen vor und nach dem Anschluss des Verbindungsstükkes an eine Blutleitung durch äusserliche Druckanwendung geschlossen werden kann.

12. Eine Vorrichtung nach Anspruch II, dadurch gekennzeichnet, dass sie ein Y-förmiges Verbindungsstück aufweist, bei dem jede Verzweigung für einen sicheren und verriegelbaren Anschluss an eine Blutleitung an die Elemente angebaut ist, und das Lumen jeder Verzweigung vor oder nach dem Anschluss an eine Butleitung durch äusserlich angewendeten Druck geschlossen werden kann.

**Revendications**

1. Un dispositif intravasculaire incluant un cathéter adapté à l'hémodialyse, équipé d'une canule (58) et comportant une section centrale (61) constituée d'une première partie d'un diamètre supérieur à celui de la partie adjacente et formant épaulement sur la section centrale faisant face à la canule, et d'une seconde partie allongée et d'un diamètre extérieur substantiellement constant, dispositif contenant une aiguille hypodermique (53) dans l'orifice de la canule et interne à la section centrale, laquelle aiguille est destinée à piquer les tissus, dispositif incluant un collier servant à empêcher la rétraction de la pointe de l'aiguille hypodermique dans l'ouverture de la canule, lequel collier comporte un corps (41) doté à son extrémité ou adjacent d'une première partie (42) munie d'un système incluant, par exemple, une paire de bras opposés ou dépendants (43, 44) associée à un ensemble comportant ladite aiguille hypodermique, au droit d'une partie de l'ensemble, proximale de la section centrale et éloignée de la pointe de l'aiguille, et, espacée de la première partie collier, une seconde partie collier s'engageant dans la deuxième partie de la section centrale pour verrouiller l'aiguille en rétraction par rapport à la canule, le dégagement de la partie collier étant rendu possible par éloignement du dispositif sans pour autant éloigner ce dernier du corps du patient une fois la piqûre effectuée, la seconde partie collier comprenant une paire de bras opposés ou dépendants (47, 48) constitués de manière à pouvoir assurer le serrage ou le relâchement de la seconde partie de la section centrale du cathéter située immédiatement devant la partie épaulée et mettre la partie épaulement en butée afin d'empêcher la rétraction de l'aiguille.

2. Un dispositif en accord avec la revendication 1 dans lequel la première et la seconde partie collier sont disposées de manière à pouvoir libérer le collier sans le désolidariser de la première partie de l'ensemble aiguille.

3. Un dispositif en accord avec la revendication 1 ou 2 dans lequel le collier est séparé de l'ensemble aiguille.

4. Un dispositif conforme à l'une des revendications suivantes et dont l'extrémité de la section centrale distale de la canule, est munie d'un dispositif permettant un branchement sur un connecteur verrouillable.

5. Un dispositif en accord avec la revendication 4 dans lequel le connecteur verrouillable comprend une chape dépassant de la face extrême de l'embout proximal de la section centrale, l'une des faces dudit moyeu faisant de préférence face à la pointe de l'aiguille pour permettre un branchement verrouillable (luer).

6. Un dispositif conforme à l'une des revendications précédentes dans lequel l'ensemble aiguille est conçu de manière à pouvoir recevoir une paire d'axes de tourillons (55) dépassant de part et d'autre de la partie de l'ensemble servant de prise manuelle et de préférence, dans lequel le collier comprend une paire de bras opposés chacun muni d'un trou d'axe permettant un montage élastique sur les axes de tourillons assurant ainsi le pivotement du collier sur la section centrale de l'ensemble aiguille.

7. Un dispositif en accord avec la revendication 6 dans lequel les axes sont situés au droit de ou adjacent à l'extrémité de ladite partie de l'ensemble aiguille proximale de la pointe de cette dernière.

8. Un dispositif en accord avec la revendication 7 dans lequel au minimum, la partie de l'ensemble aiguille contenant l'orifce du cathéter adjacente à la partie non distale est transparente de manière à signaler instantanément tout reflux.

9. Un dispositif en accord avec l'une des revendications précédentes disposant au droit de ou adjacent à l'extrémité la plus rapprochée de l'ensemble aiguille, un dispositif permettant le branchement sur un connecteur, comprenant une chape dépassant de la surface extérieure de l'extrémité proximale de l'ensemble aiguille et dont l'une des est opposée à la pointe de l'aiguille de préférence pour assurer une branchement verrouillable (luer).

10. Un dispositif en accord avec l'une des revendications précédentes dans lequel le collier comporte un embout destiné à la prise manuelle se prolongeant vers la pointe de l'aiguille, éloignée de la deuxième partie collier, cet embout disposant de nervures (52) pour éviter tout glissement lorsqu'on le saisit par le bout des doigts.

11. Un dispositif en accord avec l'une des revendications précédentes dans lequel la section centrale fait partie intégrante du système de branchement permettant de réaliser un branchement verrouillable sur une ligne de transfusion, l'orifice de ladite section centrale, laquelle est réalisée en matériau résiliant, pouvant être obturé par application d'une pression extérieure en amont et en aval du branchement de la section centrale sur une ligne de transfusion.

12. Un dispositif en accord avec la revendication 11 doté d'une section centrale de dérivation en Y dont chaque branche est massive et dotée d'un dispositif assurant un branchement verrouillable et sur sur une ligne de transfusion, l'obturation de chaque branche étant réalisée par application d'une pression externe en amont ou en aval du branchement de ladite dérivation sur une ligne de transfusion.

FIG. 1

FIG.2

FIG.3